## Europäisches Patentamt
## European Patent Office
## Office européen des brevets

(19)

(11) Veröffentlichungsnummer: **0 039 828**
**B1**

(12)

# EUROPÄISCHE PATENTSCHRIFT

(45) Veröffentlichungstag der Patentschrift:
**29.06.83**

(51) Int. Cl.³: **C 07 D 233/56**

(21) Anmeldenummer: 81103209.3

(22) Anmeldetag: **29.04.81**

(54) Verfahren zur Herstellung von 4-Methylimidazolen.

(30) Priorität: **14.05.80 DE 3018458**

(43) Veröffentlichungstag der Anmeldung:
**18.11.81 Patentblatt 81/46**

(45) Bekanntmachung des Hinweises auf die Patenterteilung:
**29.06.83 Patentblatt 83/26**

(84) Benannte Vertragsstaaten:
**CH DE FR GB IT LI NL**

(56) Entgegenhaltungen:
**EP-A-0 018 568**
**DE-A-2 360 175**
**US-A-3 715 365**

**K. HOFMANN «Imidazole and its Derivatives» Teil I 1953, INTERSCIENCE PUBLISHERS. INC., New York, Seiten 33 bis 45**

(73) Patentinhaber: **BASF Aktiengesellschaft,
Carl-Bosch-Strasse 38, D-6700 Ludwigshafen (DE)**

(72) Erfinder: **Graf, Fritz, Dr., Im Rothschild 33, D-6720 Speyer (DE)**

## Verfahren zur Herstellung von 4-Methylimidazolen

Diese Erfindung betrifft ein Verfahren zur Herstellung von 4-Methylimidazolen.

4-Methylimidazol lässt sich z.B. dadurch herstellen, dass man 1,2-Propylendiamin und Ameisensäure katalytisch zu 4-Methylimidazolin cyclisiert, welches man dann einer Wasserstoffabspaltung unterzieht. Man hat 4-Methylimidazol auch schon durch Umsetzung von Methylglyoxal oder Methylglyoxaldimethylacetal mit Formaldehyd in wässrigem Medium und in Gegenwart von Ammoniumsalzen starker Säuren hergestellt.

Bei diesem aus der US-PS 3 715 356 bekannten Verfahren führt man die Umsetzung bei pH-Werten von 7 und weniger durch. Dieses Verfahren wirft bei der technischen Durchführung Korrosionsprobleme auf und macht den Umgang mit grossen Mengen anorganischer Salzlösungen erforderlich.

Es wurde nun gefunden, dass man 4-Methylimidazole durch Umsetzung von Methylglyoxal mit Ammoniak und Aldehyden in wässriger Lösung besonders vorteilhaft herstellen kann, wenn man ein wässriges Gemisch von Methylglyoxal und dem Aldehyd entweder zu vorgelegtem wässrigem Ammoniak oder gleichzeitig mit wässrigem Ammoniak zu vorgelegtem Wasser gibt und die Umsetzung bei einem pH-Wert von über 8 durchführt.

Nach dem erfindungsgemässen Verfahren erhält man die 4-Methylimidazole in Ausbeuten bis zu 80%. Dieses vorteilhafte Ergebnis konnte aufgrund der Angaben in der US-PS 3 715 365 und in Chemical Abstracts 53 (1959) 11392a, wo angegeben ist, dass Methylglyoxal mit Ammoniak keine oder nur geringe Mengen Imidazole bilden, nicht erwartet werden. Es liess sich auch nicht aus der DE-A-23 60 175 ableiten, in der ein Verfahren zur Herstellung von in 4- und 5-Stellung unsubstituierten Imidazolen beschrieben wird, bei dem man in ein Gemisch aus dem Aldehyd und wässrigem Glyoxal, das man durch ein Reaktionsrohr leitet, Ammoniak pumpt.

Als Aldehyde können für das neue Verfahren ausser Formaldehyd auch andere Aldehyde, wie z.B. Acetaldehyd, Propionaldehyd, Benzaldehyd verwendet werden. Man erhält dann die entsprechenden in 2-Stellung substituierten Imidazole.

Die Umsetzung kann bei Temperaturen bis 150 °C durchgeführt werden. Zweckmässigerweise wählt man Temperaturen von 20 bis 100 °C. Der pH-Wert im Reaktionsgemisch muss grösser sein als 7. Vorzugsweise wird bei pH-Werten von über 8, insbesondere von 9 und darüber gearbeitet. Man setzt die Komponenten z.B. in stöchiometrischen Mengen ein, d.h. im Molverhältnis Methylglyoxal:Aldehyd:Ammoniak wie 1:1:2. Ein Überschuss von Ammoniak ist nicht von Nachteil. Ein Überschuss an Aldehyd, z.B. bis zu 100% über die stöchiometrische Menge, kann von Vorteil sein, da dadurch die Bildung von 2,4-Dimethylimidazol zurückgedrängt wird. Von wesentlicher Bedeutung ist die Reihenfolge bei der Zugabe der Komponente. Wird z.B. das Ammoniakwasser zu den vorgelegten Aldehyden gegeben, so werden nur unbefriedigende Ausbeuten erhalten.

Die nach dem erfindungsgemässen Verfahren erhältlichen 4-Methylimidazole sind wertvolle Zwischenprodukte, z.B. für die Herstellung von Pharmazeutika.

Die in den Beispielen genannten Teile und Prozente sind Gewichtseinheiten.

Beispiel 1

Eine Mischung aus 112 Teilen einer 32,4%igen wässrigen Methylglyoxallösung und 37,5 Teilen einer 40%igen wässrigen Formaldehyd-Lösung werden gleichzeitig unter Rühren und Kühlung mit 102 Teilen 25%igem wässrigen Ammoniak in 1 350 Teile vorgelegtes Wasser so eingetropft, dass die Zugabe in 30 Minuten beendet ist. Die Kühlung erfolgt so, dass sich im Reaktionsgemisch eine Temperatur von 40 °C einstellt. Der pH-Wert des Reaktionsgemisches liegt bei Beginn des Zutropfens bei 9,2 bis 9,4 und bleibt während des Reaktionsvorlaufes bei Werten über 9. Nach beendeter Zugabe wird noch ca. 30 Minuten bei 40 °C nachgerührt. Anschliessend extrahiert man 4mal mit je 500 Teilen Isobutanol. Die vereinigten Isobutanol-Extrakte werden destillativ aufgearbeitet. Man erhält 30,7 Teile 4-Methylimidazol vom Kp. 144–147 °C/1 Torr und Fp. 49 °C, entsprechend einer Ausbeute von 74,9% der Theorie, bezogen auf eingesetztes Methylglyoxal.

Beispiel 2

Ein Gemisch aus 456 Teilen einer 15,8%igen wässrigen Methylglyoxal-Lösung und 75 Teilen einer 40%igen Formaldehyd-Lösung wird während 30 Minuten unter intensivem Rühren bei 70 °C in 999 Teile einer 5,1%igen wässrigen Ammoniak-Lösung eingetropft. Der pH-Wert des Reaktionsgemisches bleibt während des Reaktionsverlaufes bei Werten über 9. Nach beendeter Zugabe wird noch ca. 30 Minuten nachgerührt. Anschliessend wird mit Isobutanol extrahiert und destillativ aufgearbeitet. Man erhält 65,2 Teile 4-Methylimidazol, entsprechend 79,2%, bezogen auf eingesetztes Methylglyoxal.

Beispiel 3

89 Teile einer 32,4%igen wässrigen Methylglyoxal-Lösung werden mit 36 Teilen Acetaldehyd gemischt und diese Mischung gleichzeitig mit 82 Teilen 25%iger wässriger Ammoniak-Lösung während 30 Minuten in 850 Teile vorgelegtes Wasser bei 40 °C unter Rühren eingetropft. Der pH-Wert des Reaktionsgemisches liegt bei Beginn des Zutropfens bei 9,2 bis 9,4 und bleibt während des Reaktionsverlaufes bei Werte über 9. Nach beendeter Zugabe wird noch 30 Minuten bei 40 °C nachgerührt. Anschliessend wird destillativ aufgearbeitet; man erhält 23,6 Teile 2,4-Dimethylimidazol, Kp 140–143 °C/1 Torr, entsprechend einer Ausbeute von 72,0%.

Beispiel 4 (Vergleichsbeispiel)

102 Teile 25%iges wässriges Ammoniak werden zu einer Lösung von 112 Teilen 32,2%igem wässrigem Methylglyoxal und 37,5 Teilen wässrigem, 40%igem Formaldehyd in 1275 Teilen Wasser unter Rühren bei 70 °C während 30 Minuten zugegeben. Es wird noch 30 Minuten bei 70 °C nachgerührt und anschliessend destillativ aufgearbeitet. Man erhält 40,5 Teile 4-Methylimidazol, entsprechend einer Ausbeute von 49,4%, bezogen auf eingesetztes Methylglyoxal.

**Patentanspruch**

Verfahren zur Herstellung von 4-Methylimidazolen durch Umsetzung von Methylglyoxal mit Ammoniak und Aldehyden in wässriger Lösung, dadurch gekennzeichnet, dass man ein wässriges Gemisch von Methylglyoxal und dem Aldehyd entweder zu vorlegtem wässrigem Ammoniak oder gleichzeitig mit wässrigem Ammoniak zu vorgelegtem Wasser gibt und die Umsetzung bei einem pH-Wert von über 8 durchführt.

**Revendication**

Procédé pour la préparation de méthyl-4 imidazoles par la mise en réaction de méthyglyoxal avec l'ammoniac et des aldéhydes en solution aqueuse, caractérisé en ce qu'on ajoute un mélange aqueux de méthylglyoxal et de l'aldéhyde, soit à une solution aqueuse d'ammoniac introduite au préalable, soit en même temps qu'une solution aqueuse d'ammoniac à de l'eau introduite au préalable et en ce que la réaction est menée à un pH de plus de 8.

**Claim**

A process for the preparation of 4-methylimidazoles by reacting methylglyoxal with ammonia and aldehydes in aqueous solution, wherein an aqueous mixture of methylglyoxal and the aldehyde is either added to aqueous ammonia already introduced into the reactor, or is added simultaneously with aqueous ammonia to water already introduced into the reactor, and the reaction is carried out at a pH above 8.